# EUROPEAN PATENT SPECIFICATION

(11) **EP 0 611 248 B1**
(45) Date of publication and mention of the grant of the patent: **22.03.2000**
(21) Application number: 94810023.5
(22) Date of filing: 14.01.1994
(51) Int. Cl.: A61K 31/505

(54) **Pharmaceutical composition for treating impotence containing an alpha-1-inhibitor and an alpha-2 inhibitor**
Zubereitungen zur Behandlung von Impotenz, die ein Alpha-1 inhibitor und ein Alpha-2 inhibitor enthalten
Compositions pharmaceutiques pour le traitement de l'impuissance, contenant un inhibiteur d'alpha-adrénocepteur et un inhibiteur d'alpha-2 adrénocepteur

(30) Priority: 10.02.1993 US 15592
(43) Date of publication of application: 17.08.1994
(73) Proprietor: B.M.R.A. CORPORATION B.V., NL-3016 DE Rotterdam (NL)
(72) Inventor: Gavras, Haralambos, Marble Head, MA 01945 (US)
(74) Representative: Fischer, Franz Josef

(56) References cited:
- EP-A- 0 408 440
- EP-A- 0 459 666
- WO-A-92/11851
- ACTA UROL BELG (BELGIUM), 1988, VOL. 56, NO. 2, PAGE(S) 272-8, Quadraccia A et al 'Yohimbine plus intracavernous vasodilators in mild vasculogenic impotence.'

## Description

The present invention relates to a pharmaceutical composition for treatment of impotency in men and method of administering same.

Functional impotence is commonly associated with hypertension and is one of the most common side effects of many antihypertensive medications. It is a major cause of non-compliance with anithypertensive treatment. Medication to enhance sexual potency in men is known, but physicians are often reluctant to prescribe such medication because its main side effect is to raise blood pressure.

Acta Urol Belg, vol. 56, no. 2, p.272-278, 1988 refers to the successful combined treatment of 20 patients with erectile failure with oral yohimbine (0,3 mg per kg daily) and weekly intracavernous injections of low dosed vasodilator drugs (papaverine and phentalamine). Although effective, self-injection under these circumstances is not readily acceptable by most patients.

WO-A-92 11851 discloses a method for treatment of sexual impotence by combining oral alpha-1 blocking agents (i.e. doxazosin or terazosin) with beta adrenergic blocking agents. It is stated that the method is suited for patients suffering from hypertension. Whereas the selective administration of either alpha or beta blocking drugs may impair the sexual function it was found, that the combination therapy with oral application was beneficial upon the sexual process. According to the experience of the inventor the combination of alpha and beta blockade does not solve the problem of sexual dysfunction.

EP-A-0 459 666 refers to the treatment of erectile impotence in a human male by administering diverse vasoactive substances, including doxazosin. Results of trials showed the satisfactory effect of an intracavernosal injection. In addition to die awkwardness and embarrassment, this procedure can lead to unpredictable results (including priapism), as well as long-term complications (scarring)

EP-A-0 408 440 describes an injectable vasoactive substances in the treatment of impotence. A typical composition contains inter alia phentolamine as alpha blocker, atropine as anticholinergic, yohimbine as alpha 2-blocker, dipyridamole as phosphodiesterase inhibitor and piribedil as dopaminergic agent and papaverine. According to this document phentolamine, a short-acting alpha-adrenergic blocker, is also used as a local vasodilator given along with other local vasodilators by intracavernosal injection on demand. Although this method is effective, it has the drawback that a self-injection is required which is not acceptable by most patients.

There is, therefore, a need for a medication that can effectively treat impotence without raising blood pressure, and similarly, a medication that can effectively treat hypertension without the resultant common side effect of impotence.

Accordingly, it is an object of the present invention to provide a pharmaceutical composition for treating impotence without causing hypertension.

Another object of the invention is to provide a pharmaceutical composition for treating impotence and hypertension.

Another object of the invention is to provide a pharmaceutical composition for treating hypertension without causing impotence.

A further object of the invention is to provide a composition for treating impotence with α-adrenoceptor inhibitors according to the definition of claim 1 having antihypertensive effect.

These and other objects of the invention will become apparent in the light of the accompanying disclosure.

These and other objects of the present invention are achieved by providing a pharmaceutical composition comprising a combination of active agents consisting of an effective amount for treating impotency of an effective amount of an α₂-adrenoceptor inhibitor and an α₁-adrenoceptor inhibitor having antihypertensive characteristics according to the definition of claim 1.

The sexual function is governed by the central and peripheral autonomic nervous system and is impaired in patients with autonomic dysfunction. Central α₂-adrenoceptors are crucial in the regulation of this function by changing the level of sympathetic outflow. Stimulation of the presynaptic central α₂-adrenoceptors exerts a sympathoinhibitory effect, leading to impotence. A typical example of this is the antihypertensive drug clonidine, a central α₂-agonist with excellent antihypertensive effect associated very frequently with impotence. Inhibition of these same receptors with the α₂-antagonist yohimbine is known to enhance sexual function by disinhibiting the central sympathoinhibitory neurons and increasing the sympathetic outflow to the periphery, which almost invariably raises also the blood pressure. Hence, use of yohimbine is restricted for fear of hypertensive cardiovascular complications. Prazosin is a peripheral α₁-adrenoceptor antagonist which causes vasodilation and lowers blood pressure by inhibiting the action of catecholamines on the vascular α₁-adrenergic receptors. It is a common antihypertensive agent and has also been used locally by intracorporeal injection for treatment of functional impotence, with proven success, but limited applicability, because of the need and timing of parenteral administration.

Vasoconstriction in response to elevated catecholamines is mediated mostly via stimulation of peripheral α₁-adrenoceptors, and partly about 20%, via stimulation of peripheral post-synaptic α₂-adrenoceptors in the vascular wall. Blockade of either type of peripheral α-adrenoceptors by a specific inhibitor causes fall of blood pressure. Thus, the α₂-antagonist yohimbine acting peripherally would also produce some blood pressure lowering, but this effect is far outweighed by its action on the central presynaptic α₂-adrenoceptors that increase the sympathetic outflow to the periphery and cause intense vasoconstriction mediated via the overriding stimulation of peripheral α₁-adrenoceptors. Hence, when the two drugs are used in combination, the low dose prazosin would prevent the yohimbine-induced blood pressure rise, whereas the high dose prazosin would produce an antihypertensive effect, and both would allow the additional small peripheral blood pressure lowering effect of yohimbine itself to become manifest.

Yohimbine has a central α₂-blocking effect, that results in disinhibition of central sympathoinhibitory neurons and causes a hyperadrenergic state that raises blood pressure via stimulation of peripheral vasopressor α₁-adrenoceptors. With the combined use of prazosin, however, these α₁-receptors are already blocked. Yohimbine also has a blocking effect on peripheral vascular α₂-adrenoceptors, that are also vasopressor, although less so than the vascular α₁-receptors. This accounts for the drop of blood pressure below that expected for prazosin alone. Yohimbine is also known to have a parasympathetic agonistic effect, that accounts for the lack of increase, or slight decrease, in heart rate and for the beneficial effect on sexual impotence via its action on the arterioles and venules of the penile corpora cavernosa. From these interactions, it is apparent that the combination of yohimbine and prazosin has the unique properties of specifically enhancing cavernosal erectile function despite increased central sympathetic outflow, maintaining a status of balanced sympathetic-parasympathetic influence on the heart, inhibiting peripheral vasopressor α₁- and α₂-adrenergic vascular receptors. Varying the dose of prazosin administered such that it is lower in normotensive men and higher in hypertensive men, maintains the blood pressure or induces a decrease in systemic blood pressure.

The following example is provided solely as a non-restrictive illustration of the effect described above and to provide a clearer understanding of the advantage of the invention.

Clinical studies were conducted on hypertensive men having impotence associated with antihypertensive treatment. Prazosin, 5 mg, taken orally three times daily, reduced blood pressure without resolving the impotence. Addition of yohimbine, 5 mg, to the prazosin, 5 mg, taken orally three times daily, reduced blood pressure an additional 2-5 mmHg below that resulting from prazosin alone, without a change in heart rate, and additionally resolved the impotence.

### Clinical test 1

46 White male, long-standing mild hypertension controlled on various β-blockers, latest metoprolol 50 mg qd. Impotence with all of them. On stopping metoprolol BPs (BP = blood pressure) rose to 150/100 range, impotence resolved only partially. Started prazosin, increasing to 5 mg tid (= three times per day), impotence unchanged. Added yohimbine 5 mg tid, impotence completely resolved, BPs remained in the 130/80 range.

### Clinical test 2

50 black male, long-standing essential hypertension, controlled on hydrochlorothiazide (HCTZ) 50 mg qd. Saw urologist for impotence, had normal testosterone levels, yet given monthly I.M. injection of depot-testosterone without effect on impotence. Stopped both above medications, impotence continued, testosterone levels normal. Started prazosin, increasing to 5 mg tid plus yohimbine 5 mg tid, impotence resolved.

### Clinical test 3

48 white male moderately severe hypertension, on multiple drug therapy including ACE inhibitor, methyldopa and HCTZ, complained of impotence. Stopped methyldopa and HCTZ, continued enalapril and added prazosin, up to 10 mg tid with some improvement in erectile capacity, but insufficient. Added yohimbine 10 bid with resolution of impotence while maintaining good BP control.

### Clinical test 4

63 white male mild hypertension controlled on various drugs given as monotherapy in the past, but impotent with each one. Started doxazosin, increasing to 8 mg qd with no change in impotence. Added yohimbine 5 mg tid, impotence resolved.

### Clinical test 5

38 black male hypertension controlled on verapamil and HCTZ, impotence which resolved partially on stopping both drugs. Started on prazosin increasing to 10 mg tid, no change in impotence, inadequate BP control. Added yohimbine 5 mg tid, then 10 mg tid, BP further decreased, impotence resolved.

### Comparison test

64 Hispanic male with severe hypertension on multiple drugs in the past, total impotence; stopped all drugs on his own and impotence resolved but BP rose to 220/120. Placed on verapamil and doxazosin increasing to 16 mg qd, BP 150/80-90 impotence returned. Added yohimbine 5 mg tid, then 10 mg tid, BP slightly decreased but impotence persisted. Reports that when he stopped everything again for one week impotence resolved but he had pounding headaches. Apparently poor blood flow due to partial arterial obstruction causes impotence on lower BP.

## Claims

1. A pharmaceutical composition for treatment of impotency in men by oral administration characterised in that it comprises a combination of active agents consisting of
a) prazosin, doxazosin or terazosin as an α₁-adrenoceptor inhibitor having antihypertensive characteristics; and
b) yohimbine as an α₂-adrenoceptor inhibitor.

2. The pharmaceutical composition according to claim 1 wherein prazosin, doxazosin or terazosin is comprised in the range from 1 mg to 10 mg per dosage unit.

3. The pharmaceutical compostion according to claim 1 or 2 wherein yohimbine is comprised in the range from 5 mg to 10 mg per dosage unit.

4. The pharmaceutical composition according to claim 1 wherein the α₁-adrenoceptor is prazosin.

5. The pharmaceutical composition according to claim 4 comprising 5 mg yohimbin per dosage unit.

6. The pharmaceutical composition according to claim 4 comprising 10 mg yohimbin per dosage unit.

7. The pharmaceutical composition according to claim 5 or 6 comprising 1 mg, 5 mg or 10 mg prazosin per dosage unit.

8. The pharmaceutical composition according to claim 5 or 6 comprising 1 mg or 4 mg doxazosine per dosage unit.

9. The pharmaceutical composition according to claim 5 or 6 comprising 1 mg or 5 mg terazosin per dosage unit.

## Patentansprüche

1. Pharmazeutische Zusammensetzung für die Behandlung von Impotenz von Männern durch eine orale Verabreichung, dadurch gekennzeichnet, dass sie eine Kombination von aktiven Komponenten enthält, welche aus
a) Prazosin, Doxazosin oder Terazosin als adrenergischer α₁-Rezeptorhemmer mit antihypertensiven Eigenschaften; und
b) Yohimbin als adrenergischer α₂-Rezeptorhemmer
besteht.

2. Pharmazeutische Zusammensetzung gemäss Anspruch 1, worin das Prazosin, Doxazosin oder Terazosin in einer Menge im Bereich von 1 mg bis 10 mg pro Dosiseinheit enthalten ist.

3. Pharmazeutische Zusammensetzung gemäss Anspruch 1 oder 2, worin Yohimbin in einem Anteil im Bereich von 5 mg bis 10 mg pro Dosiseinheit enthalten ist.

4. Pharmazeutische Zusammensetzung gemäss Anspruch 1 worin der adrenergischer α₁-Rezeptorhemmer Prazosin ist.

5. Pharmazeutische Zusammensetzung gemäss Anspruch 4, enthaltend 5 mg Yohimbin pro Dosiseinheit.

6. Pharmazeutische Zusammensetzung gemäss Anspruch 4, enthaltend 10 mg Yohimbin pro Dosiseinheit.

7. Pharmazeutische Zusammensetzung gemäss Anspruch 5 oder 6, enthaltend 1 mg, 5 mg oder 10 mg Prazosin pro Dosiseinheit.

8. Pharmazeutische Zusammensetzung gemäss Anspruch 5 oder 6, enthaltend 1 mg oder 4 mg Doxazosin pro Dosiseinheit.

9. Pharmazeutische Zusammensetzung gemäss Anspruch 5 oder 6, enthaltend 1 mg oder 5 mg Terazosin pro Dosiseinheit.

## Revendications

1. Composition pharmaceutique pour le traitement de l'impuissance chez les hommes par administration orale, caractérisée en ce qu'elle comprend une combinaison d'agents actifs consistant en
a) prazosine, doxazosine ou térazosine en tant qu'inhibiteur des adrénorécepteurs α_{1,} ayant des caractéristiques antihypertensives; et
b) en yohimbine en tant qu'inhibiteur des adrénorécepteurs α₂.

2. Composition pharmaceutique selon la revendication 1, dans laquelle le contenu de prazosine, doxazosine ou térazosine est de l'ordre de 1 mg à 10 mg par unité de dosage.

3. Composition pharmaceutique selon la revendication 1 ou la revendication 2, dans laquelle le contenu de yohimbine est de l'ordre de 5 mg à 10 mg par unité de dosage.

4. Composition pharmaceutique selon la revendication 1, dans laquelle l'inhibiteur des adrénocepteurs α₁ est la prazosine.

5. Composition pharmaceutique selon la revendication 4, comprenant 5 mg de yohimbine par unité de dosage.

6. Composition pharmaceutique selon la revendication 4, comprenant 10 mg de yohimbine par unité de dosage.

7. Composition pharmaceutique selon la revendication 5 ou la revendication 6, comprenant 1 mg, 5 mg ou 10 mg de prazosine par unité de dosage.

8. Composition pharmaceutique selon la revendication 5 ou la revendication 6, comprenant 1 mg ou 4 mg de doxazosine par unité de dosage.

9. Composition pharmaceutique selon la revendication 5 ou la revendication 6, comprenant 1 mg ou 5 mg de térazosine par unité de dosage.
